# EUROPEAN PATENT APPLICATION

(11) **EP 3 923 294 A1**
(43) Date of publication of application: **15.12.2021**
(21) Application number: 20179500.2
(22) Date of filing: 11.06.2020
(51) Int. Cl.: G16H 30/20, G16H 40/63, G16H 50/30

(54) **PATIENT CRITICAL CONDITION DETECTION**

(71) Applicant: Koninklijke Philips N.V., 5656 AG Eindhoven (NL)
(72) Inventor: VOGTMEIER, Gereon, 5656 AE Eindhoven (NL); SREENIVASAN, Rithesh, 5656 AE Eindhoven (NL); OKALY, Vikram, 5656 AE Eindhoven (NL); JOHNSON, Mark, 5656 AE Eindhoven (NL)
(74) Representative: Philips Intellectual Property & Standards

(57) **Abstract**

This disclosure provides a system (100) for patient critical condition detection for an at least semi-autonomous medical imaging procedure. It comprises at least one data processing unit (110), and at least one vital parameter monitoring unit (120), configured to monitor at least one vital parameter of a patient (P) during the medical imaging procedure. The at least one data processing unit (110) is configured to detect, based on at least obtained monitoring data of the at least one vital parameter monitoring unit (120), a critical condition of the patient (P) that indicates an active intervention. Further, the at least one data processing unit (110) is configured to initiate at least one counteraction to the detected critical condition of the patient (P).

## Description

### FIELD OF THE INVENTION

The present invention relates to medical imaging. In particular, the present invention relates to a system or device for patient critical condition detection for an at least semi-autonomous medical imaging procedure, a, preferably, computer-implemented method for patient critical condition detection for an at least semi-autonomous medical imaging procedure, and a computer program element.

### BACKGROUND OF THE INVENTION

Especially during an at least semi-autonomous imaging, situations may occur where a patient might be alone in the preparation phase, the actual scanning or imaging phase as well as after the actual scanning or imaging. This could make a patient nervous or frightened. In an unfavorable case, this could lead to an impairment of the patient's health.

There is also a possibility of adverse reactions to medications or aids, such as contrast media, for example, injected intravenously during a procedure. For example, reactions to intravenously injected contrast media are observed in 5 % to 8 % of patients who receive them. Some of the reactions may be mild reactions include a feeling of warmth, nausea, and vomiting. Generally, these symptoms occur only for a short period of time and do not require treatment. Moderate reactions, including severe vomiting, hives, and swelling, are observed to occur in 1 % of patients receiving contrast media and frequently require treatment. Severe, life-threatening reactions, including anaphylaxis, occur in 0.1 % of people receiving contrast media, with an expected death rate of one person in every 75.000.

In case a critical medical situation occurs it may take some time before staff support is available and a first health stabilizing procedure will help the patient for the first time.

### SUMMARY OF THE INVENTION

There may, therefore, be a need to further improve patient critical condition detection for medical imaging. The object of the present invention is solved by the subject-matter of the independent claims, wherein further embodiments are incorporated in the dependent claims.

According to a first aspect, there is provided a system for patient critical condition detection for an at least semi-autonomous medical imaging procedure. The system comprises:
- at least one data processing unit, and
- at least one vital parameter monitoring unit, configured to monitor at least one vital parameter of a patient during the medical imaging procedure,
- wherein the at least one data processing unit is configured to detect, based on at least obtained monitoring data of the at least one vital parameter monitoring unit, a critical condition of the patient that indicates an active intervention and/or treatment, and
- wherein the at least one data processing unit is configured to initiate a counteraction to the detected critical condition of the patient.

In this way, the system monitors the patient's vital signs and/or other parameters to detect a critical condition and/or an adverse reaction, and provides and/or initiates a counteraction to the detected critical condition and/or an adverse reaction. This may comprise an at least semi-automated escalation sequence or the like. This can ensure a significantly faster treatment or care of the patient. In a favorable case, the medical imaging procedure does not have to be interrupted or only interrupted for a short time, so that in addition to patient safety, efficiency of the medical imaging procedure can also be increased. Overall it supports an autonomous medical imaging procedure implementation.

The above system may be applied to, optionally at least partly incorporated in a medical imaging system, which may at least comprise a medical imaging device, or may be provided separately.

As used herein, the one or more data processing units may be formed as a suitable computer means, which may comprise one or more of a processor, a memory, one or more data interfaces, one or more communication interfaces, etc. There may be two or more data processing units, which may be distributed over different systems, including e.g. a medical imaging system, a clinical or hospital computer system, or the like. Likewise, the functions as described herein may be distributed over different data processing units.

The one or more vital parameter monitoring units may comprise, for example, may be formed as or may comprise different sensors configured to detect and/or monitor relevant vital parameters or indicators of the patient.

The term critical condition or critical situation referring to the patient may be broadly understood. It may comprise a wide range of situations in which the patient may be found, e.g. a feeling of anxiety, physical impairments such as those of the cardiovascular system, respiration, etc., allergic reactions, etc. A particularly critical situation, especially if it can be harmful to health or life-threatening, may be referred to as an emergency.

The term counteraction may also be broadly understood. It may comprise any suitable action which aims to support the patient in the situation and to help the patient out as much as possible. For example, the counteractions may range from simple adjustment of the patient's position, e.g. via an adjustable patient table, to an automatic injection of medicine or medication, and a call of emergency doctors, etc., wherein individual counteractions together may form an escalation chain in which an individual counteraction form a corresponding stage. The stages of the escalation chain and/or their order may depend on the seriousness of the critical situation and/or a determined risk level of the patient as described below. By way of example, the severity of the stages is preferably ascending, so that simpler counteractions are initiated first, and these are then increased when indicated. One of the upper or latter stages may be, for example, an emergency call, an automatic stop of the medical imaging procedure, etc. The one or more counteractions may at least partly be performed manually by staff, computer-aided and/or at least semi-automatically or fully automatically. The one or more counteractions do not necessarily have to be performed directly on the patient, but can also comprise an alarm, such as a communication and/or message to a medical professional, such as imaging staff and/or a medical doctor or the like, and/or an emergency call, etc.

From a functional perspective, the data processing unit may receive input data from various vital parameter monitoring units, such as sensors, on patient vitals, such as oxygen saturation, heart rate, blood pressure, camera feedback. Based on this, the data processing unit may apply one or more computing algorithms to detect a critical situation, especially an emergency. This detection may be based on e.g. abnormal and/or changing vital signs, and may comprise e.g. hypotension, severe hypertension, bradycardia, tachycardia, arrhythmias and respiratory distress, which are indicators that the patient is unstable and might crash. In such critical conditions or situations, the system may initiate one or more of the counteractions, optionally along the escalation chain. Optionally, in some situations, the system may automatically stop the medical imaging procedure and may initiate an emergency procedure, also referred to as code blue. Optionally, to ensure that a e.g. a medical doctor will be available in a defined time interval, the system may track necessary emergency support options. For example, in case a medical doctor would not be available in the hospital to consult the patient within a maximum allowed time delay, then the medical imaging procedure for the patient may be put on hold until all conditions for a minimized risk are fulfilled. Further optionally, schedule adaptation and additional optimization may be part of the mitigation procedure. Optionally, the medical imaging procedure may be reshuffled to adapt to the patient's condition, wherein an automatic stop of the medical imaging procedure and/or remote and/or staff support calls may be initiated in high risk situations. Optionally, relevant data may be logged or recorded, which may help to ensure full documentation. These data may, optionally, also be used for a continuously improved risk management based on all historic treatments and observed situations. Further optionally, in less critical situations, other methods may be sufficient to stabilize the patient, comprising, for example, include the lifting and/or lowering of parts of the patient table and/or bringing the patient into a different position. These options are also available as the module can communicate with the imaging system and/or patient table and/or patient transport system. Optionally, other means for communication with the patient may be provided and may differ from each other depending on the patient profile and/or an estimated most efficient way.

The functions of the system described herein, especially when performed by the data processing unit, may, for example, be implemented in the form of a computer program and may also be referred to as modules.

Optionally, the counteraction is further based on an obtained patient profile. This profile may comprise, for example, records of existing complaints, protocols from previous (imaging) examinations, health data, treatment data, past medical history of the patient, a current medical condition of the patient, etc.

For example, prior to the medical imaging procedure, the patient may take part in a registration process, where, for example, a questionnaire may be filled in, the health condition of the patient may be determined, etc. These details may also be obtained from an electronic medical record (EMR), wherein an EMR may comprise a medical and treatment history of the patient in one practice, and/or an electronic health record (HER), wherein a HER may comprise information about the total health of the patient, which may also be cross-practice or cross-institutional, or other sources for patient profile information.

According to an embodiment, the at least one data processing unit may further be configured to determine, based on at least an obtained patient profile and/or the obtained monitoring data, a level of risk of the specific patient associated with one or more precautionary measures and/or the at least one counteraction, and an escalation chain of the at least one counteraction being adaptable or is adapted on the basis of the determined level of risk.

For example, the one or more precautionary measures may comprise a pre-administration of a medicine or medication, a certain position of the patient, a support of vital parameters by medical devices, the provision of staff or medical professionals, or the like.

Optionally, based on the determined risk level, the patient may then be classified as, for example: high risk, moderate risk, low risk, optionally with additional information about special attention points. Note that variations and more graduated risk levels may be optional. The risk classification may be performed based on the obtained patient profile and/or the obtained monitoring data, and may consider various parameters, such as patient diagnosis, present vital parameters, Glasgow coma scale (GCS) score, history of allergies, whether the patient is already on any support systems like ventilator, an ET tube with Ambubag, pressor, etc. For example, in MRI, history of pacemakers or any other devices that are not compatible with magnetic energy are to be elicited and test to be avoided in case if the MRI system is not compatible with such devices. With regard to the administration of a contrast medium, a history of renal disease and/or a present status of renal parameters like creatinine may also be considered. The specific risk level may, for example, be taken into account when providing personnel or medical professionals, such as that at a high risk level a medical doctor should be available in the medical imaging department itself, at a moderate risk level a medical doctor should be available in the hospital or nearby, and at a low risk level a medical doctor should be available remotely to consult or guide the management of occurring situations.

Based on the determined risk level, the escalation chain may be adapted, e.g. individual stages can be skipped or prioritized.

In an embodiment, the at least one data processing unit may further be configured to determine, based on the determined level of risk of the patient, and prior to a medical imaging phase of the medical imaging procedure, presence and/or reachability requirements of a medical professional as one counteraction. For example, depending on the risk level or the above risk classification of the patient, it may be determined whether the patient can perform fully autonomous medical imaging due to a low risk level, or whether staff or medical professionals have to be available remotely on demand, as may be the case with a moderate risk level, or whether staff or medical professionals have to necessarily be on site, as is the case with a high risk level.

According to an embodiment, the determined level of risk may be adapted depending on the obtained monitoring data. This allows an early reaction to an imminent situation, possibly even preventive.

In an embodiment, the one or more counteractions may comprise one or more of: controlling an actuator to change a position and/or orientation of the patient, communicating with the patient to mitigate or eliminate the critical condition of the patient, contacting a medical professional via a communications means to consult the patient on site or remotely, administering medication to the patient, reshuffling the medical imaging procedure, controlling operation of a medical imaging system, and stopping operation of a medical imaging system.

For example, the actuator may be configured to drive an adjustable patient table, or the like. This allows the patient to be placed in an upright position, for example, or in a lying position, etc. The communication with the patient to mitigate or eliminate the critical condition of the patient may comprise a loudspeaker or headphone announcement, a screen message or screen instructions, etc. Contacting a medical professional and/or imaging staff or the like may comprise a message and/or alarm, preferably generated automatically, transmitted e.g. via a telephone or computer network. This may be transmitted to a computer means, such as a portable device, a smartphone, or the like. Thus, an autonomous medical imaging procedure implementation can be supported. Further, patient safety can be improved.

Further, by way of example, the following counteractions may be applied:
In case of hypotension: pressor medicine like dopamine infusion may be administered along with foot end elevation.

In case of mild allergic reaction: a bolus antihistamine injection and, if it is more severe, bolus steroid injection may be administered. In case of a high severe anaphylactic reaction, epinephrine bolus or an infusion may be administered.

In case of bradycardia: bolus injection of Atropine and, if severe, bradycardia or cardiac arrest bolus or infusion of epinephrine may be administered.

In case of cardiac arrhythmias: bolus injection of antiarrhythmic medicine or arrhythmias causing cardiac arrest, epinephrine and need DC shock defibrillator or CPR may be administered or applied.

In case of hypotension due to a contrast medium or dehydration: a saline infusion may be administered.

In case of pulmonary edema: the patient made to sit, a bolus injection of diuretics may be administered, and a fluid infusion may be avoided.

In case of hypoxia: an oxygen inhalation may be given.

In case of excessive blood pressure, due to reaction or some other cause: beta blockers, an NTG infusion, or short and rapid acting antihypertensive medications may be administered.

In an embodiment, the system may further comprise at least one medication dispensing unit, wherein the at least one data processing unit is configured to control the at least one medication dispensing unit to administer at least one medication associated with the counteraction to the detected critical condition to the patient.

For example, the one or more medication dispensing units may comprise an electronically controllable delivery unit, such as a pump, a nebulizer, etc., a medication reservoir, an interface to the patient, etc. It may, for example, already be connected to the patient in advance and be controlled if required. Thus, as one counteraction, medication or medicine can be administered automatically so that there is little or no delay in taking the counteraction. This is a risk minimization driven approach to have short reaction times for the patient care.

According to an embodiment, the at least one data processing unit may further be configured to determine, based on at least an obtained patient profile and/or the detected critical condition of the patient, an appropriate dosage of the at least one medication to be administered to the patient.

In an embodiment, the at least one data processing unit may further be configured to control the at least one medication dispensing unit to administer a first dose of the at least one medication, to first administer a first dose of the medication, subsequently to obtain a measurement of the patient response to the first dose and/or critical condition, and subsequently, depending on the measurement, to administer at least a second dose of the medication when indicated. The measurement may be performed by using, for example, the at least one vital parameter monitoring unit. Thus, the risk of the patient may be further decreased. Thus, medication is based on a feedback measurement under consideration of a temporal change of the patient's condition.

According to an embodiment, the at least one data processing means may further be configured to administer the at least one medication to the patient via one or more of injection and inhaling. Note that other forms of administration may be applied, as long as they can preferably be controlled electronically. This allows the administration of medication to be automated, thus saving further time for patient care.

In an embodiment, the at least one vital parameter monitoring unit may comprise one or more of: an oxygen saturation monitoring means, a heart rate monitoring means, a blood pressure monitoring means, a camera means configured to capture the patient for, preferably automatic, image analysis by the at least one data processing means.

According to an embodiment, the at least one data processing unit may further be configured to control a medical imaging system to automatically stop when the critical condition of the patient is detected. For this purpose, the data processing unit may be connected to or may form at least a part of a control system of the medical imaging system. Parallel to this, an emergency call or the like may also be made. Thus, patient safety is further improved.

In an embodiment, the automatic stopping may be configured multi-stage, and in a first stage it is verified whether the counteraction has eliminated the critical condition or situation or at least sufficiently mitigated it, and in a subsequent stage the automatic stop is initiated if the counteraction is or was not sufficient. Thus, patient safety is further improved.

According to an embodiment, the at least one data processing unit may further be configured to determine and/or monitor whether a presence and/or reachability of a medical professional meets or still meets estimated or expected timing conditions of presence and/or reachability, and to schedule or re-schedule the medical imaging procedure depending on the determination and/or monitoring. For example, staff or medical professionals may be, preferably electronically, tracked, or they report their current or intended location on their own initiative, or this information is taken from a duty roster or the like. This information may form input data for the at least one data processing unit and may then be processed. Further, the at least one data processing unit may be configured to contact or communicate with the medical professional in order to ensure the presence and/or reachability. In case that the estimated or expected timing conditions are not to be met, the at least one data processing unit may send a signal, control data, or the like to schedule or re-schedule the medical imaging procedure. Thus, patient safety is further improved.

In an embodiment, the at least one data processing unit may further be configured to automatically contact or communicate with a medical professional via at least one communication means in response to the detection of the critical condition of the patient. For example, it may send a signal, a message, or the like via a telephone, mobile and/or computer network to be received by a corresponding communication means, such as a telephone, a terminal, a computer, another portable device, or the like. Thus, even an emergency call can be performed in an automatic manner, saving time for patient care.

According to an embodiment, the at least one data processing unit may further be configured to verify, during a preparation phase of the patient preceding a medical imaging phase of the medical imaging procedure, whether the at least one vital parameter monitoring unit is ready for operation in such a way that it is actually able to monitor the at least one vital parameter of the patient. For example, the at least one data processing unit may monitor the status of at least one vital parameter monitoring unit and reacts if they are not operational or malfunctioning. Thus, patient safety is further improved.

According to a second aspect, there is provided a method for patient critical condition detection for an at least semi-autonomous medical imaging procedure. The method comprises the steps of:
- obtaining, from at least one vital parameter monitoring unit, at least one vital parameter of a patient during the medical imaging procedure,
- detecting, by at least one data processing unit, and based on at least obtained monitoring data of the at least one vital parameter monitoring unit, a critical condition of the patient that indicates an active intervention, and
- initiating, by the at least one data processing unit, at least one counteraction to the detected critical condition of the patient.

For example, the method may be carried out by using the system described above with respect to the first aspect.

In this way, the monitors the patient's vital signs and/or other parameters to detect a critical condition and/or an adverse reaction, and provides and/or initiates a counteraction to the detected critical condition and/or an adverse reaction. This may comprise an at least semi-automated escalation sequence or the like.

According to another aspect, there is provided a computer program element controlling one or more of the devices and systems as described above which, if the computer program element is executed by a data processing unit or processor, is adapted to perform one or more of the functions and/or methods as described above.

According to another aspect, there is provided a computer-readable medium having stored the computer program element as described above.

The computer program element can, for example, be a software program but can also be a FPGA, a PLD or any other appropriate digital means.

It should be noted that the above embodiments may be combined with each other irrespective of the aspect involved. Accordingly, the method and/or use may be combined with structural features and, likewise, the system may be combined with features described above with regard to the method and/or use. Advantageously, the benefits provided by any of the above aspects equally apply to all of the other aspects and vice versa.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig 1 shows in a schematic overview a system for patient critical condition detection for an at least semi-autonomous medical imaging procedure according to an embodiment.
Fig. 2 shows in block diagram an operating principle of a system for patient critical condition detection for an at least semi-autonomous medical imaging procedure according to an embodiment.
Fig. 3 in a flow chart a computer-implemented method for patient critical condition detection for an at least semi-autonomous medical imaging procedure according to an embodiment.

### DETAILED DESCRIPTION OF EMBODIMENTS

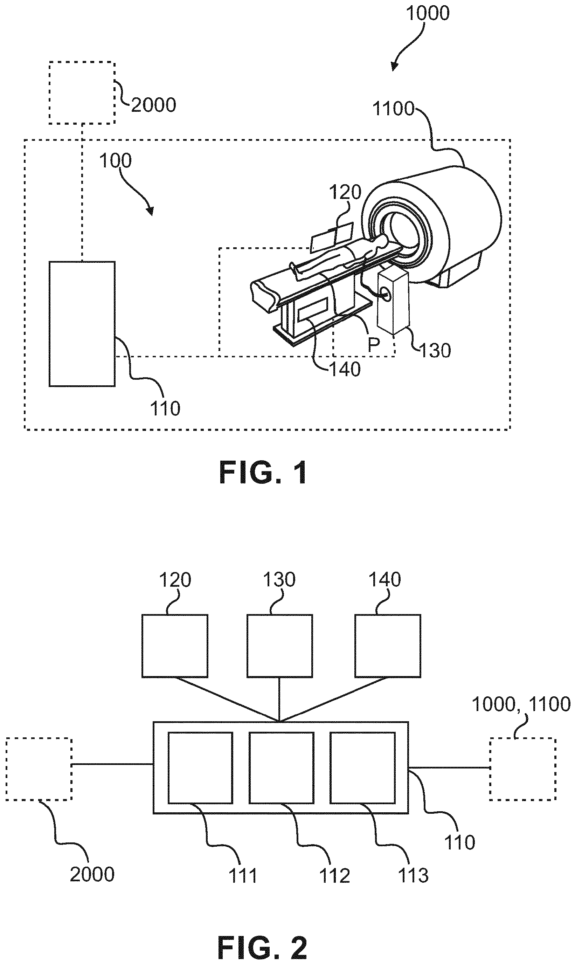

Fig. 1 shows in a schematic overview a system 100 that is configured for patient critical condition detection for an at least semi-autonomous medical imaging procedure that is applied to a patient P. The medical imaging procedure is, for example, carried out by using a medical imaging system 1000, which comprises at least a medical imaging device 1100, which is here, by way of example, represented as a Magnetic Resonance image (MRI) imaging device. It is noted that the medical imaging device 1100 may also be provided as a Computer Tomography (CT) imaging device, and/or a 3D-rotational X-ray (3DRX) imaging device, or another medical imaging device.

The system 100 may be used in preparation of and during operation of the medical imaging system 1000, in particularly during operation of the medical imaging device 1100. It can be integrated into it or provided separately.

The system 100 comprises at least one data processing unit 110 and at least one vital parameter monitoring unit 120. In at least some embodiments, the system 100 further comprises at least one medication dispensing unit 130. Further, in at least some embodiments, the system 100 comprises at least one actuator device 140.

Although only one data processing unit 110 is shown here, two or more may be provided, in particular the medical imaging system 1000 and/or the medical imaging device 1100 may optionally comprise its own data processing unit and/or control system. Likewise, the functions of the system 100 described herein may be carried out in a distributed manner using two or more data processing units.

As indicated in Fig. 1, the data processing unit 110 is operatively connected to the at least one vital parameter monitoring unit 120, to the at least one medication dispensing unit 130, and to the at least one actuator 140. Further, the data processing unit 120 is, for example, a suitable computer means, which may comprise one or more of a processor, a memory, one or more data interfaces, one or more communication interfaces, etc. Further, in at least some embodiments, the data processing unit 110 is operatively connected to a data source 2000, where, for example, patient information is stored. The data source 2000 is, for example, part of a medical imaging facility and/or hospital computer system, or the like. The patent information may comprise patient data, a patient profile or the like from an electronic medical record (EMR), wherein an EMR may comprise a medical and treatment history of the patient in one practice, and/or an electronic health record (HER), wherein a HER may comprise information about the total health of the patient, which may also be cross-practice or cross-institutional.

The at least one vital parameter monitoring unit 120 is generally configured to detect and/or monitor at least one vital parameter of the patient P during the medical imaging procedure. It may comprise one or more sensors or other measurement means suitable for detection and/or monitoring of a respective vital parameter of the patient P. In at least some embodiments, the at least one vital parameter monitoring unit 120 comprises one or more of an oxygen saturation monitoring means, a heart rate monitoring means, a blood pressure monitoring means, a camera means that configured to capture the patient for image analysis by the at least one data processing means, or other suitable sensor and/or monitoring means. Although Fig. 1 exemplary shows two vital parameter monitoring units 120, there may be provided only one or a plurality of two, three, four or more, depending on the specific requirements of patient P. The at least one vital parameter monitoring unit 120 may be connected or applied to the patient P by using a suitable patient interface that depends on the measurement and/or monitoring technique used. The at least one vital parameter monitoring unit 120 may be at least partly controlled by or may at least provide at least one vital parameter of the patient P to the at least one data processing unit 110.

The at least one medication dispensing unit 130 is generally configured to administer or provide a medication or medicine to the patient P. For this purpose, it may comprise or may be connected to a suitable patient interface, such as an intravenous port, a respiratory mask, or the like. Further, the at least one medication dispensing unit 130 may comprise an electronically controllable delivery device, such as a pump, a nebulizer, or the like, to deliver the medication or medicine from a reservoir or the like to the patient P. The at least one medication dispensing unit 130 may be at least partly controlled by the at least one data processing unit 110.

The at least one actuator device 140 is generally configured to change a position and/or orientation of the patient P. For this purpose, it may comprise, for example, one or more motors, drives, or the like. As indicated in Fig. 1, the at least one actuator device 140 may be provided at a patient table, a patient transport system, or the like. There, for example, the inclination, height, a sitting position, a lying position or the like may be adjusted by the at least one actuator device 140. It may be at least partly controlled by the at least one data processing unit 110.

Fig. 2 shows in block diagram an operating principle of the system 100. The at least one data processing unit 110 is configured to at least partly control the operation of the system 100, which comprises the above at least one vital parameter monitoring unit 120, the at least one medication dispensing unit 130, and the at least one actuator device 140. Fig. 2 further shows the data source 2000 and the medical imaging system 1000 or medical imaging device 1100 respectively, wherein the at least one data processing unit 110 is configured to at least receive data, e.g. patient information, from the data source 2000 and is further configured to at least partly control the medical imaging system 1000 and/or medical imaging device 1100.

The at least one data processing unit 110 carries out functions or functional modules of the system 100, which may be implemented by, for example, a computer program that may be stored in the memory, etc. As indicated in Fig. 2, there is a first module 111, which may also referred to as a risk assessment and/or risk minimization module, a second module 112, which may also referred to as a critical condition or situation detection module, and a third module 113, which may also be referred to as a critical condition or situation medication dispensing module.

Generally, the at least one data processing unit 110 is configured to detect, based on at least obtained detection and/or monitoring data of the at least one vital parameter monitoring unit 120, a critical condition of the patient P that indicates an active intervention. Further, it is configured to initiate at least one counteraction to the detected critical condition of the patient P.

In at least some embodiments, the at least one data processing unit 110 is further configured to determine, based on obtained patient profile data and/or the obtained monitoring data, a level of risk of the specific patient associated with one or more precautionary measures and/or the at least one counteraction, and an escalation chain of the at least one counteraction being adaptable or is adapted on the basis of the determined level of risk.

In at least some embodiments, the at least one data processing unit 110 is further configured to determine, based on the determined level of risk, and prior to a medical imaging phase of the medical imaging procedure, presence and/or reachability requirements of a medical professional as one counteraction.

In at least some embodiments, the determined level of risk is adapted depending on the obtained monitoring data.

In at least some embodiments, the counteraction comprises one or more of: controlling the actuator device 140 to change a position and/or orientation of the patient P, communicating with the patient P to mitigate or eliminate the critical condition of the patient, contacting a medical professional via a communications means to consult the patient on site or remotely, administering medication to the patient P, reshuffling the medical imaging procedure, controlling operation of a medical imaging system, and stopping operation of the medical imaging system 1000.

In at least some embodiments, the at least one data processing unit 110 is configured to control the at least one medication dispensing unit 130 to administer at least one medication associated with the counteraction to the detected critical condition to the patient.

In at least some embodiments, the at least one data processing unit 110 is further configured to determine, based on at least an obtained patient profile and/or the detected critical condition of the patient P, an appropriate dosage of the at least one medication to be administered to the patient P.

In at least some embodiments, the at least one data processing unit 110 is further configured to control the at least one medication dispensing unit to administer a first dose of the at least one medication, to first administer a first dose of the medication, subsequently to obtain a measurement of the patient response to the first dose and/or critical condition, and subsequently, depending on the measurement, to administer at least a second dose of the medication when indicated.

In at least some embodiments, the at least one data processing means 110 is further configured to administer the at least one medication to the patient via one or more of injection and inhaling.

In at least some embodiments, the at least one data processing unit 110 is further configured to control the medical imaging system 1000 to automatically stop when the critical condition of the patient is detected.

In at least some embodiments, wherein the automatic stopping is multi-stage, and in a first stage it is verified whether the counteraction has eliminated the situation or at least sufficiently mitigated it, and in a subsequent stage the automatic stop is initiated if the counteraction is not sufficient.

In at least some embodiments, the at least one data processing unit 110 is further configured to determine, prior to a medical imaging phase of the medical imaging procedure, and based on at least an obtained patient profile, a level of risk of the patient associated with presence and/or reachability requirements of a medical professional. The patient profile may result from a patient registration procedure, comprising e.g. a questionnaire, examinations, or the like, or may be provided by e.g. data source 2000.

In at least some embodiments, the at least one data processing unit 110 is further configured to determine and/or monitor whether a presence and/or reachability of a medical professional meets estimated or expected timing conditions of presence and/or reachability, and to schedule or re-schedule the medical imaging procedure depending on the determination and/or monitoring.

In at least some embodiments, wherein the at least one data processing unit 110 is further configured to automatically contact a medical professional via at least one communication means in response to the detection of the critical condition of the patient.

In at least some embodiments, the at least one data processing unit 110 is further configured to verify, during a preparation phase of the patient preceding a medical imaging phase of the medical imaging procedure, whether the at least one vital parameter monitoring unit 120 is ready for operation in such a way that it is actually able to monitor the at least one vital parameter of the patient P. This can also be referred to as an initial and possibly also continuous function test.

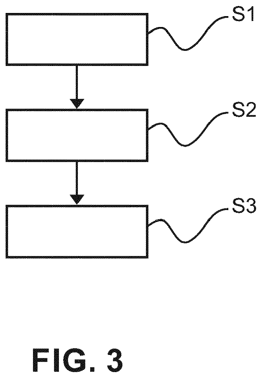

Fig. 3 shows in a flow chart a computer-implemented method for patient critical condition detection for an at least semi-autonomous medical imaging procedure. The method may be carried out by the above system 100 and/or the medical imaging system 1000.

In a step S1, the data processing unit 110 obtains at least one vital parameter of the patient P from the at least one vital parameter monitoring unit 120 during the medical imaging procedure.

In a step S2, the at least one data processing unit 110 detects based on at least obtained monitoring data of the at least one vital parameter monitoring unit 120, a critical condition of the patient P that indicates an active intervention.

In a step S3, the at least one data processing unit 110 initiates at least one counteraction to the detected critical condition of the patient.

The following detailed embodiments refer to the first module 111, which may also referred to as a risk assessment and/or risk minimization module, the second module 112, which may also referred to as a critical condition or situation detection module, and the third module 113, which may also be referred to as a critical condition or situation medication dispensing module. These embodiments provide further details on how the system 100 for patient critical condition detection for an at least semi-autonomous medical imaging procedure, and the method for patient critical condition detection for an at least semi-autonomous medical imaging procedure can be realised as would be appreciated by the skilled person.

### Embodiment 1: Risk assessment and/or risk minimization module 111

The first module 111 is configured to:
First, a risk calculation and/or stratification is carried out. Before the actual medical imaging procedure, the patient P may take part in a registration procedure. For example, a questionnaire may be filled in by the patient or attender depending on the patient condition. These or further details may also be obtained from the data source 2000, e.g. utilizing EMR/HER or other sources for patient profile information.

The patient P is then classified as, for example: high risk, moderate risk, low risk with additional information about special attention points.

The risk classification is performed based on parameters, such as a patient diagnosis, present vital parameters, Glasgow coma scale (GCS) score, a history of allergies, whether the patient P already needs any support system, such as a ventilator, ET tube with Ambubag, pressors etc. For MRI, history of pacemakers or any other devices that are not compatible with magnetic energy may be elicited and test to be avoided in case if the MRI system is not compatible therewith. When a contrast medium is to be given, a history of renal disease and/or a present status of renal parameters, such as creatinine.

Further, risk mitigation is carried out. For a high risk patient, medical professionals should be available in the imaging department or near the medical imaging system 1000 during the procedure. For a moderate risk patient, medical professionals should be available in the hospital or nearby. For a low risk patient, medical professionals should be available remotely to guide the management of complications and/or critical situations.

Further, risk minimization and continuous monitoring is carried out. In the preparation phase, the patient P gets an intravenous port in place for administering medication, such as emergency medication. Further, an oxygen source, e.g. a cylinder, may be provided. The risk minimization module is further configured to check if the at least one vital parameter monitoring unit 120 (e.g. for detecting and/or monitoring HR, BP, SpO2, CO2, and RR) is connected to the patient before starting the medical imaging procedure. For example, it may be checked the data inflow from the at least one vital parameter monitoring unit 120 for sensor quality. Further, the module may initiate a test with the patient P for allergic reaction using a minimal dose of the medication. Optionally, a risk ranking may be adapted according to the scan planning and sequence. The risk ranking may also be based on the patient's position.

When starting the actual medical imaging procedure the first module receives inputs data from the at least one vital parameter monitoring unit 120, wherein based on an emergency condition detected by the second module 112, which may also referred to as a critical condition or situation detection module, one or more counteractions may be performed by controlling the at least one medication dispensing unit 130. The module continuously monitors the status of the at least one vital parameter monitoring unit 120 and the patient's vital parameters. Further, it considers an interaction with the system 100 for the progress of the patient P and performs communication to a medical professional and/or care taker if required.

### Embodiment 2: critical condition or situation detection module 112

The second module 112, i.e. critical condition or situation detection module may receive input data from at least one vital parameter monitoring unit 120 on patient vital parameter, such as oxygen saturation, heart rate, blood pressure, camera feedback and then applies one or more computational steps, e.g. algorithms, to detect a critical condition. For example, an abnormal vital sign to be detected may include hypotension, severe hypertension, bradycardia, tachycardia, arrhythmias and respiratory distress, which may be an indicator that the patient is unstable and might crash. In some or all critical situations the system may be automatically stopped and an e.g. emergency procedure, e.g. code blue, may be initiated. To ensure that medical professionals are available in a defined time interval, critical condition or situation detection module may track counteraction options. For example, if medical professionals would not be available in the hospital to be at the patient within the maximum allowed time delay, the medical imaging procedure for the patient would put on hold until all conditions for min. risk are fulfilled. Further, schedule adaptation and additional optimization may be performed. Optionally, reshuffling of the medical imaging procedure may be carried out to adapt to the patient's condition. Further optionally, the medical imaging procedure may be automatically stopped. As a further option, remote and/or staff support calls may be initiated, particularly in high risk situations. Further, relevant data may be logged to ensure documentation and/or to create data for continuously improving the risk management based on historic treatments and observed situations. In less critical situations, other methods might be sufficient to stabilize the patient. This may include the lifting and/or lowering of parts of the patient table and/or bringing the patient into a different position. These options are also available as the module can communicate with the medical imaging system 1000 and/or the actuator device 140 arranged at the patient table, patient transport system etc.

### Embodiment 3: critical condition or situation medication dispensing module 113

The critical condition or situation medication dispensing module obtains input data from the Risk assessment and/or risk minimization module, and controls dispensing of the appropriate medication. For example, depending on the status and the patient, the module controls to dispense first a lower dose to measure the patient reaction and to optimize the final dose based on the feedback measurement under consideration of the temporal change of the patient's condition.

It has to be noted that embodiments of the invention are described with reference to different subject matter. In particular, some embodiments are described with reference to method type claims whereas other embodiments are described with reference to device or system type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 1000: medical imaging system
- 1100: medical imaging device
- 100: system
- 110: data processing unit
- 111: first module
- 112: second module
- 113: module
- 120: vital parameter monitoring unit
- 130: medication dispensing unit
- 140: actuator device
- S1-S5: method steps

## Claims

1. A system (100) for patient critical condition detection for an at least semi-autonomous medical imaging procedure, comprising:
- at least one data processing unit (110), and
- at least one vital parameter monitoring unit (120), configured to monitor at least one vital parameter of a patient (P) during the medical imaging procedure,
- wherein the at least one data processing unit (110) is configured to detect, based on at least obtained monitoring data of the at least one vital parameter monitoring unit (120), a critical condition of the patient (P) that indicates an active intervention, and
- wherein the at least one data processing unit (110) is configured to initiate at least one counteraction to the detected critical condition of the patient (P).

2. The system according to claim 1, wherein the at least one data processing unit (110) is further configured to determine, based on obtained patient profile and/or the obtained monitoring data, a level of risk of the specific patient associated with one or more precautionary measures and/or the at least one counteraction, and an escalation chain of the at least one counteraction being adaptable or is adapted on the basis of the determined level of risk.

3. The system according to claim 2, wherein the at least one data processing unit (110) is further configured to determine, based on the determined level of risk, and prior to a medical imaging phase of the medical imaging procedure, presence and/or reachability requirements of a medical professional as one counteraction.

4. The system according to claim 2 or 3, wherein the determined level of risk is adapted depending on the obtained monitoring data.

5. The system according to any one of the preceding claims, wherein the counteraction comprises one or more of: controlling an actuator (140) to change a position and/or orientation of the patient, communicating with the patient to mitigate or eliminate the critical condition of the patient, contacting a medical professional via a communications means to consult the patient on site or remotely, administering medication to the patient, reshuffling the medical imaging procedure, controlling operation of a medical imaging system, and stopping operation of a medical imaging system.

6. The system according to any one of the preceding claims, further comprising at least one medication dispensing unit (130), wherein the at least one data processing unit (110) is configured to control the at least one medication dispensing unit to administer at least one medication associated with the counteraction to the detected critical condition to the patient.

7. The system according to claim 6, wherein the at least one data processing unit (110) is further configured to determine, based on at least an obtained patient profile and/or the detected critical condition of the patient (P), an appropriate dosage of the at least one medication to be administered to the patient.

8. The system according to claim 6 or 7, wherein the at least one data processing unit (110) is further configured to control the at least one medication dispensing unit (130) to administer a first dose of the at least one medication, to first administer a first dose of the medication, subsequently to obtain a measurement of the patient response to the first dose and/or critical condition, and subsequently, depending on the measurement, to administer at least a second dose of the medication when indicated.

9. The system according to any one of claims 6 to 8, wherein the at least one data processing means (110) is further configured to administer the at least one medication to the patient via one or more of injection and inhaling.

10. The system according to any one of the preceding claims, wherein the at least one vital parameter monitoring unit comprises one or more of: an oxygen saturation monitoring means, a heart rate monitoring means, a blood pressure monitoring means, a camera means configured to capture the patient for image analysis by the at least one data processing means.

11. The system according to any one of the preceding claims, wherein the at least one data processing unit (110) is further configured to control a medical imaging system (1000) to automatically stop when the critical condition of the patient is detected.

12. The system according to any one of the preceding claims, wherein the at least one data processing unit (110) is further configured to determine and/or monitor whether a presence and/or reachability of a medical professional meets estimated or expected timing conditions of presence and/or reachability, and to schedule or re-schedule the medical imaging procedure depending on the determination and/or monitoring.

13. The system according to any one of the preceding claims, wherein the at least one data processing unit (110) is further configured to automatically contact a medical professional via at least one communication means in response to the detection of the critical condition of the patient.

14. A method for patient critical condition detection for an at least semi-autonomous medical imaging procedure, comprising:
- obtaining, from at least one vital parameter monitoring unit (120), at least one vital parameter of a patient during the medical imaging procedure,
- detecting, by at least one data processing unit (110), and based on at least obtained monitoring data of the at least one vital parameter monitoring unit, a critical condition of the patient that indicates an active intervention, and
- initiating, by the at least one data processing unit (110), at least one counteraction to the detected critical condition of the patient.

15. A computer program element for controlling a system according to any of claims 1-13, which when executed by a processor is configured to carry out the method of claim 14.
